# EUROPEAN PATENT SPECIFICATION

(11) **EP 2 310 084 B1**
(45) Date of publication and mention of the grant of the patent: **23.03.2016**
(21) Application number: 09739944.8
(22) Date of filing: 01.05.2009
(51) Int. Cl.: A61N 1/05

(54) **ELECTRODE LEAD SYSTEM**
ELEKTRODENLEITUNGSSYSTEM
SYSTÈME DE FIL D ÉLECTRODE

(30) Priority: 02.05.2008 US 49927 P
(43) Date of publication of application: 20.04.2011
(73) Proprietor: Medtronic, Inc., Minneapolis, MN 55432-5604 (US)
(72) Inventor: BONDE, Eric, H., Minnetonka MN 55345 (US); TESTERMAN, Roy, L., New Hope MN 55427 (US); HERBERT, Timothy, P., Maple Grove MN 55311 (US); CHRISTOPHERSON, Mark, A., Shoreview MN 55126 (US)
(74) Representative: Noble, Nicholas
(86) International application number: PCT/US2009/042546
(87) International publication number: WO 2009/135142

(56) References cited:
- WO-A-2008/048471
- US-A1- 2005 096 710
- US-A1- 2008 103 570
- US-A1- 2008 172 116
- US-B1- 7 248 930
- ELEANOR V GOODALL ET AL: "Position-Selective Activation of Peripheral Nerve Fibers with a Cuff Electrode" IEEE TRANSACTIONS ON BIOMEDICAL ENGINEERING, IEEE SERVICE CENTER, PISCATAWAY, NJ, US, vol. 43, no. 8, 1 August 1996 (1996-08-01), XP011006271 ISSN: 0018-9294

## Description

### TECHNICAL FIELD

The present disclosure relates generally to an implantable simulation system for stimulating and monitoring soft tissue in a patient, and more particularly, the present disclosure relates to a lead system for positioning an electrode and lead body of an implantable stimulation system within a body.

### BACKGROUND

Sleep apnea generally refers to the cessation of breathing during sleep. One type of sleep apnea, referred to as obstructive sleep apnea (OSA), is characterized by repetitive pauses in breathing during sleep due to the obstruction and/or collapse of the upper airway, and is usually accompanied by a reduction in blood oxygenation saturation.

One treatment for obstructive sleep apnea has included the delivery of electrical stimulation to the hypoglossal nerve, located in the neck region under the chin. Such stimulation therapy activates the upper airway muscles to maintain upper airway patency. In treatment of sleep apnea, increased respiratory effort resulting from the difficulty in breathing through an obstructed airway is avoided by synchronized stimulation of an upper airway muscle or muscle group that holds the airway open during the inspiratory phase of breathing. For example, the genioglossus muscle is stimulated during treatment of sleep apnea by a cuff electrode place around the hypoglossal nerve.

Because of the significant amount of movement in multiple directions that can take place under the chin, positioning an electrode to enable stimulation of the hypoglossal nerve becomes a significant challenge. On the one hand, placement of the electrode and lead in close proximity to the hypoglossal nerve can result in irritation to the nerve as a result of normal motion of the chin and neck, while on the other hand, without close adherence to the nerve, buildup of connective tissue between the nerve and the electrode cuff and lead can occur, causing high thresholds, thereby reducing the effectiveness of the delivered stimulation by the device. Similarly, a related challenge in maintaining the proper positioning of the electrode includes making a proper placement of a lead body that extends from the electrode to an implantable pulse generator, which is typically located in the pectoral region of the patient.

WO 2008/048471 is directed to devices, systems and methods for nerve stimulation for OSA therapy.

### SUMMARY

The present invention is defined by the appended claims. The examples, embodiments, or aspects of the present description that do not fall within the scope of said claims are merely provided for illustrative purposes and do not form part of the invention. Furthermore, the methods presented in the present description are provided for illustrative purposes only and do not form part of the present invention.

### BRIEF DESCRIPTION OF THE DRAWINGS

Aspects and features of the present invention will be appreciated as the same becomes better understood by reference to the following detailed description of the embodiments of the present disclosure when considered in connection with the accompanying drawings, wherein:
FIG. 1 is a schematic diagram of an implantable stimulation system, according to an embodiment of the present disclosure;
FIG. 2 is a side view of a lead system utilized in an implantable stimulation system, according to an embodiment of the present disclosure;
FIG. 3 is a perspective view of an expandable cuff electrode, according to an embodiment of the present disclosure;
FIG. 4 is a sectional view of the cuff electrode as taken along lines 4 - 4 of Figure 3, according to an embodiment of the present disclosure;
FIG. 5 is a sectional view of an expandable electrode cuff in a larger diameter, partially fully engaged position, according to an embodiment of the present disclosure;
FIG. 6 is a side plan view of a lead assembly including a cuff electrode and a lead body, according to an embodiment of the present disclosure; and
FIG. 7 is plan view schematically illustrating deployment of the lead assembly of FIG. 6, according to an embodiment of the present disclosure.

### DESCRIPTION OF EMBODIMENTS

The following detailed description is merely exemplary in nature and is not intended to limit the present disclosure or the application and uses of the embodiments of the present disclosure. Furthermore, there is no intention to be bound by any expressed or implied theory presented in the preceding technical field, background, brief summary or the following detailed description.

Embodiments of the present disclosure provide a lead system including a cuff electrode configured to be secured on a nerve and a lead body extending from the cuff electrode to connect with an implantable pulse generator located remotely from the location of the nerve. In one embodiment, the lead body includes a first lead portion, an anchor, and a second lead portion. The anchor is secured relative to a non-nerve anatomical structure, such as a tendon, in close proximity to the location of the cuff electrode on the nerve. In one aspect, the first lead body is configured with a length substantially greater than a distance between the secured cuff electrode and the secured anchor. In another aspect, the first lead portion includes a generally serpentine configuration formed of a resilient material.

With this arrangement, a strain relief mechanism is established to prevent the cuff electrode from being dislodged from the nerve, or from placing undue stress on the nerve, on the cuff electrode, and/or on the lead body. By securing the anchor to a non-nerve anatomical structure near the secured cuff electrode and by providing a relatively long first lead portion with a generally serpentine configuration, the lead system provides a generous length of the first lead portion between the anchor and the cuff electrode, thereby ensuring that little, if any, strain is exerted on the lead body, on the cuff electrode, and/or on the nerve.

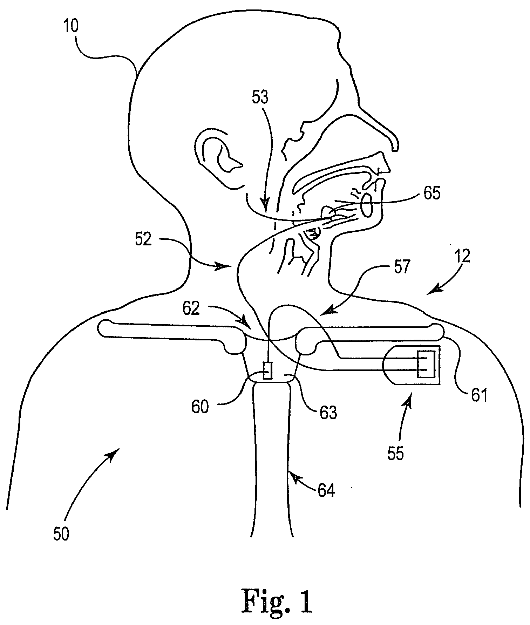

These embodiments, and other embodiments, will be described in association with FIGS. 1-7.

FIG. 1 is a schematic diagram of an implantable stimulation system 12, according to an embodiment of the present disclosure. As illustrated in FIG.1, an example of an implantable stimulation system 12 according to one embodiment of the present disclosure includes an implantable pulse generator (IPG) 55, capable of being surgically positioned within a pectoral region of a patient 10, and a stimulation lead 52 electrically coupled with the IPG 55 via a connector (not shown) positioned within a connection port of the IPG 55. The lead 52 includes an electrode or electrode system 65 and extends from the IPG 55 so that the electrode system 65 is position around a desired nerve, such as the hypoglossal nerve 53 of the patient 10, to enable stimulation of the nerve 53, as described below in detail. An exemplary implantable stimulation system in which lead 52 may be utilized, for example, is described in U.S. Patent No. 6,572,543 to Christopherson et al., and further includes a sensor lead 57 electrically coupled to the IPG 55 and extending from the IPG 55 so that a sensor or transducer 60 can be positioned in the patient 10 for sensing of respiratory effort.

The sensor 60 may be a pressure sensor that is surgically implanted in a region that has pressure continuity with the intrapleural space, such as the suprasternal notch, the space between the trachea and esophagus, or by being attached to either of the trachea or esophagus. The sensor 60 may also be positioned intercostally, or secured in a position for sensing pressure at the posterior side of the manubrium. The suprasternal notch 62 and manubrium 63 of the sternum 64 are well known structures on the upper chest that are in anatomical continuity with the intrapleural space. It is also well known that changes in intrapleural pressure provide a characteristic respiratory effort waveform.

The location for placement of the sensor 60 is, at least in part, chosen as a function of a delay, i.e. the propagation time associated with a pressure waveform characteristic of respiratory effort propagating from the respiratory point of origin to the sensor position. The chosen location is also a function of the amount of filtering necessary to achieve a usable sensed signal at a particular location, i.e. the amount of filtering that is necessary to remove waveforms other than the waveform associated with the desired sensed characteristic, such as the filtering required to remove cardiac waveform activity, for example. The positioning of the sensor 60 enables the IPG 55 to receive respiratory effort waveform information utilized to determine increased respiratory effort, which is then used by the IPG 55 to control delivery of therapy in response to determined increases in respiratory effort.

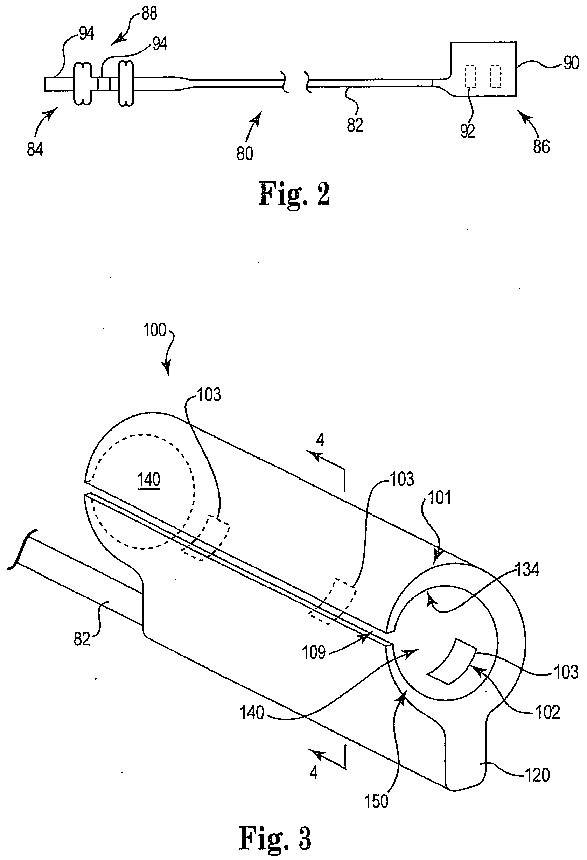

FIG. 2 is a side view of a lead system 80 utilized in the implantable stimulation system, according to an embodiment of the present disclosure, with lead system 80 being used in place of lead 52 of FIG. 1. In one embodiment, as illustrated in FIG. 2, the lead system 80 includes a lead body 82, connector 88, and an expandable cuff electrode 90. The lead body 82 extends from a proximal end 84 to a distal end 86, with the connector 88 being positioned at the proximal end 84 of the lead body 82 for electrically connecting the lead body 82 to the IPG 55. The expandable electrode cuff 90 is located at the distal end 86 of the lead body 82 and is configured to be positioned around a target nerve, such as a hypoglossal nerve or other nerve. The electrode cuff 90 includes one or more electrodes 92 embedded within a wall of the electrode cuff 90 so that when the electrode cuff 90 is positioned around the nerve, the respective electrodes 92 are in contact with the nerve. In one aspect, the lead body 82 includes conductors (not shown) extending within the lead body 82 to electrically connect the electrodes 92 and the connector 88 so that the electrodes 92 are electrically coupled to the IPG 55 via respective connector pins 94 of the connector 88, as is known in the art.

FIG. 3 is a perspective view of an expandable electrode cuff 100, according to one embodiment of the present disclosure, which can be deployed in place of the cuff electrode 90 in lead system 80. As illustrated in FIG. 3, cuff 100 includes a cuff body 101 and an array 102 of electrodes 103. In general terms, the cuff body 101 defines a lumen 140 through which a target nerve will extend. Among other features, the cuff body 101 includes a pair of resilient fingers 134, 150 (e.g., flange members) that have a generally arcuate shape and that extend from a base portion 120 of cuff body 101. In general terms, by pulling the ends of the resiliently, biased fingers 134,150 apart from each other, access to lumen 140 is provided for engaging a target nerve. Upon release of the fingers 134, 150, the cuff body 101 resumes the shape illustrated in FIG. 3. As will be further described later, once positioned on a nerve, the materials and construction of the fingers 134, 150 permit automatic expansion of the size of lumen 140 to accommodate expansion of the size of the nerve encircled by the cuff body 101. In this manner, the electrodes 103 are held in close contact against the nerve while allowing for expansion of a diameter of lumen 140 defined between fingers 134, 150 and base 120.

In one embodiment, the array 102 of electrodes 103 are embedded within a wall of the cuff body 101 with the respective electrodes 103 spaced apart from each other along a length of the cuff body 101. In some embodiments, the electrodes 103 are aligned in series along a single longitudinal axis on a common side or portion of the cuff body 101.

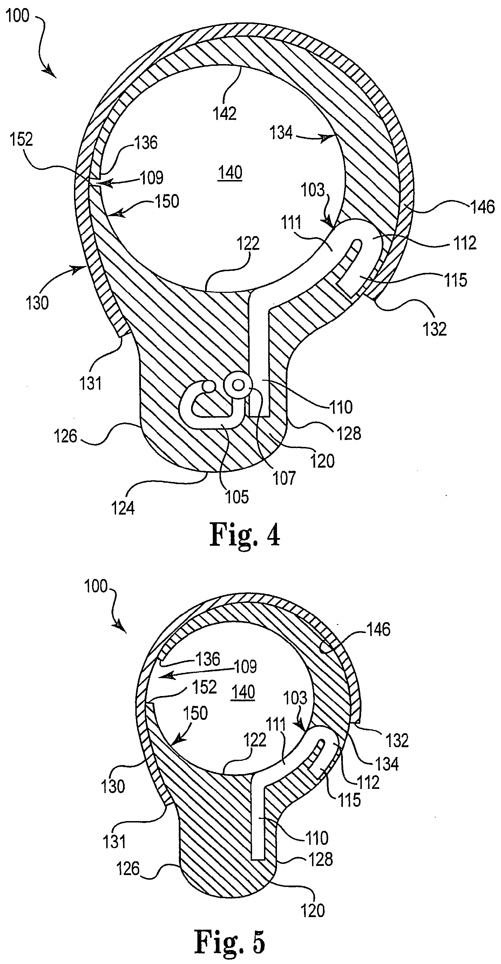

In some embodiments, cuff 100 additionally includes an outer flap or flange member that is biased and configured to maintain releasable coverage of at least a portion of an outer surface of the cuff body 101 and of the re-closable opening 109 between the distal ends of fingers 134, 150, as will be described in more detail in association with at least Figures 4-5. However, in other embodiments, this outer flap is omitted. In either case, Figure 3 illustrates cuff body 101 without an outer flap for illustrative clarity in viewing the features and attributes of cuff body 101 that would otherwise be obscured by the presence of an outer flap.

FIG. 4 is a sectional view of the expandable electrode cuff 100, as taken along lines 4-4 of FIG. 3, according to an embodiment of the present disclosure. As illustrated in FIG. 4, in one embodiment the cuff body 101 of the expandable electrode cuff 100 includes a base portion 120, a first flange member 130, a second flange member 134, and a third flange member 150.

FIG. 4 also illustrates one of the electrodes 103 that is embedded in cuff body 101, as previously illustrated in FIG. 3. As illustrated in FIG. 4, electrode 103 includes a proximal trunk portion 110, a body portion 111, and a distal hook portion 112 with the distal hook portion 112 defining a recess 114 that extends between a distal tip portion 115 and body portion 111. The proximal trunk portion 110 extends within, and is embedded within, the base portion 120 of the cuff body 101 while the body portion 111 is embedded within the second flange member 134 to be exposed within lumen 140.

As illustrated in FIG. 4, the base portion 102 includes a top wall 122, a bottom wall 124, a first side wall 126, and a second side wall 128. The second flange member 134 and the third flange member 150 comprise arcuate shaped fingers that are shaped, biased, and have a length to define a generally circular shaped lumen 140. The second flange member 134 extends generally outward from the second side wall 128 and from the top wall 122 of the base portion 120 with at least a portion of the second flange member 134 being spaced apart from the top wall 122 of the base portion 120. On the other hand, the third flange member 150 extends generally outward from the first side wall 126 and from the top wall 122 of the base portion 120.

In some embodiments, the first flange member 130 includes a proximal end 131 and a distal end or portion 132 with the proximal end 131 being bonded to the third flange member 150 along a portion of an outer wall 152 of the third flange member 150. The first flange member 130 has a length sufficient to extend about, and be in releasable contact with, the periphery or outer surface of both the third flange member 150 and a majority of a length of the second flange member 134. In this arrangement, with the first flange member 130 extending coextensively with (and in releasable contact with) a majority of the arcuate length of the second flange member 134, the first flange member 130 is biased to maintain coverage across the substantially re-closable opening 109 (between the distal ends 136, 152 of the respective second and third flange members 134, 150).

In one aspect, the free distal portion (including distal end 132) of the first flange member 130 extends in a first direction or orientation opposite to the direction (or orientation) in which the free distal portion (including distal end 136) of the second flange member 134 extends. In this arrangement, with the first flange member 130 and the second flange member 134 being biased in an overlapping, releasably contacting relationship, each of the distal ends 132, 136 of the respective first and second flange members 130, 134 will move in opposite directions upon expansion of the lumen 140 in response to a swollen nerve or during positioning of the cuff 100 about a nerve, as will be further described in association with at least FIG. 5.

During its normal, unbiased state, prior to insertion around the nerve, the electrode cuff 100 is in a fully engaged position (shown in FIG. 4). Together, the distal ends 136, 152 of the respective second and third flange members 134, 150 form the substantially re-closable opening 109 that provides selective access to lumen 140 and that generally maintains a nerve securely within the lumen 140, unless affirmative steps are taken to remove the electrode cuff 100 from the nerve.

Depending on the size of the nerve about which the cuff 100 is mounted, the electrode cuff 100 may be either in the fully engaged position of FIG. 4 or in a partially fully engaged position (FIG. 5), which occurs in the event that the cuff automatically expands in size to accommodate a larger sized diameter nerve or a swollen nerve. In the partially fully engaged position illustrated in FIG. 5, the general configuration of the electrode cuff 100 remains generally the same as the fully engaged position except that, because the first flange member 130 is slidably movable relative to the second flange member 134 and because the generally flexible, distal portion of the second flange member 134 permits rotational movement of the second flange member 134 away from the third flange member 150, the distal end 132 of the first flange member 130 will be positioned along the outer side wall 146 of the second flange member 134 in a location spaced further away from the second side wall 128 than shown in FIG. 4. Likewise, upon rotation of the second flange member 134 away from the third flange member 150, the substantially re-closable opening 109 between the distal end 136 of the second flange member 134 and the distal end 152 of the third flange member 150 will at least partially open as a gap between the distal ends 136, 152, as shown in FIG. 5. Nevertheless, despite this gap, in the partially fully engaged position the lumen 140 remains generally closed because the first flange member 130 has a length sufficient to still extend across the gap (in the substantially re-closable opening 109) between the respective distal ends 136, 152 and to extend further over (and in releasable contact with) substantial remaining portions of the second flange member 134.

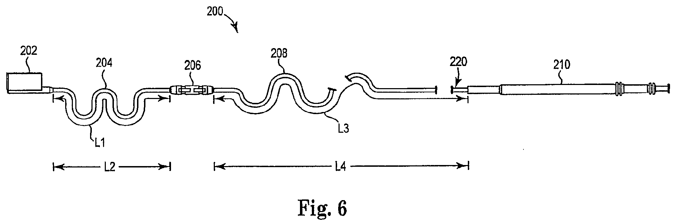

FIG. 6 is a side plan view of a lead system, according to an embodiment of the present disclosure. As illustrated in FIG. 6, in one embodiment, the lead system 200 comprises a cuff electrode 202, a first lead portion 204, a second lead portion 208, an anchor 206, and a connector 210. In one embodiment, the cuff electrode 202 of lead system 200 comprises a cuff electrode having substantially the same features and attributes as the embodiments of cuff electrode 100, as previously described in association with FIGS. 3-5. However, in other embodiments, the cuff electrode 202 comprises other features and attributes of conventional electrode cuffs.

In one aspect, the first lead portion 204 extends proximally from the cuff electrode 202 and is formed of a resilient material to have a pre-formed, generally serpentine configuration. In another aspect, the second lead portion 208 also is formed of a resilient material to have a pre-formed, generally serpentine configuration. However, the second lead portion 208 has a length substantially greater than a length of the first lead portion 204. For example, in one non-limiting example, the second lead portion 208 has a length about seven times the length of the first lead portion 204. In this arrangement, the second lead portion 208 has a length sufficient to extend from the placement location of the cuff electrode 202 at a target nerve (e.g. hypoglossal nerve) to the location of placement of the IPG 55 (FIG. 1) within a pectoral region.

In one embodiment, the generally serpentine configuration of the respective first and second lead portions 204, 208 comprises a generally sinusoidal pattern including a series of S-shaped curves. However, in other embodiments, the generally serpentine configuration of the respective first and second lead portions 204, 208 comprises other undulating or curvaceous shapes and patterns. In some embodiment, a respective one of the first and second lead portions 204, 208 has a first type of undulating or curvaceous pattern while the other respective one of the first and second lead portions 204, 208 has a second, different type of undulating or curvaceous pattern.

In one aspect, the anchor 206 is interposed between the first lead portion and the second lead portion 208 and is configured to be secured relative to a body structure adjacent to the target nerve on which the cuff electrode 202 is mounted. The connector 210 extends proximally from the second lead portion 208 and is configured to electrically connect to an implantable pulse generator (such as IPG 55 in FIG. 1). Accordingly, in this embodiment, the first anchor 206 is not accompanied by a second anchor along the lead body.

However, in some embodiments, the lead system 200 additionally includes a second anchor interposed between the second lead portion 208 and the connector 210 at a location represented by reference numeral 220 (or another location closer to the first anchor 206). This second anchor is configured to be secured relative to a body structure and is provided to provide strain relief for the lead system relative to the IPG 55 and relative to the first anchor 206 (when it is secured in close proximity to the cuff electrode and nerve).

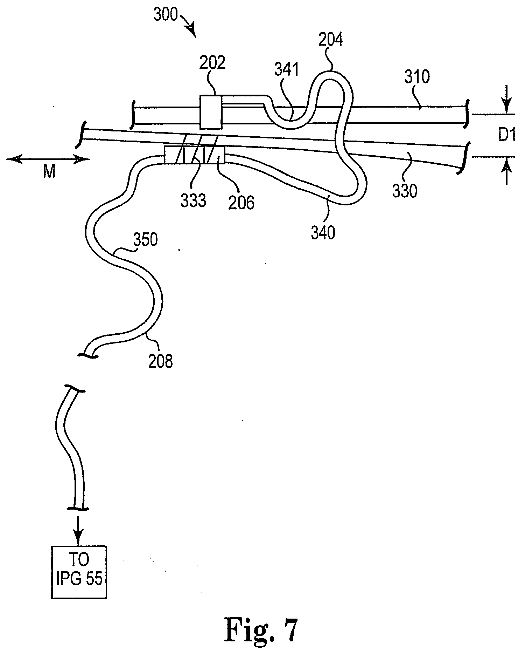

FIG. 7 schematically illustrates a method 300 of placing or delivering the lead assembly 200. In this method, the method 300 comprises first providing a lead assembly such as lead assembly 200 as previously described in association with FIG. 6. After releasably securing the cuff electrode 202 about a hypoglossal nerve 310 (or other target nerve), the first lead portion 204 is maneuvered and arranged to extend proximally from the cuff electrode 202 to a non-nerve bodily structure 330 (such as a digastric tendon, other tendon, or bony structure) that is in close proximity to the hypoglossal nerve 310 and through the mylohyoid muscle (not shown). The first anchor 206 is secured (via sutures 333 or other biocompatible fasteners) to the non-nerve bodily structure 330 adjacent the target nerve 310 while maintaining at least a portion 341 of the first lead portion 204 in a generally serpentine or undulating configuration between the cuff electrode 202 and the anchor 206. In this arrangement, other portions 340 of the first lead portion 204 no longer retain their pre-formed generally serpentine shape.

In some embodiments, method 300 includes arranging the secured cuff electrode 202 and the secured anchor 206 in substantially the same vertical plane such that a majority of the first lead portion 204 extends generally perpendicular to the vertical plane. In one aspect, this arrangement helps to provide the strain relief described below in more detail.

The second lead portion 208 is then maneuvered and arranged to extend proximally from the anchor toward an implantable pulse generator, using tunneling tools as known in the art. Finally, the connector is used to establish electrical communication and mechanical connection to the IPG 55.

In this arrangement, by securing the anchor 206 onto a non-nerve anatomical structure 330 in close proximity to the location at which the cuff electrode 202 is secured onto the target nerve 310, a substantial length or portion 341 of the first lead portion 204 remains in a serpentine or otherwise undulating configuration to thereby provide strain relief upon relative movement of the nerve 310 and the tendon 330. In other words, upon movement of the cuff electrode 202 as the nerve 310 moves, only minimal strain or no strain is placed on the lead portion 204, on the cuff electrode 202, and/or on the nerve 310 because of the position of the nearby anchor 206 and because of the combination of the sufficient length and generally serpentine configuration of the first lead portion 204 (which ensures that a substantial room for movement is allowable for the cuff electrode 202).

In one embodiment, as illustrated in FIG. 6, the first lead portion 204 has an arcuate length (L1) of 7.62 cm (three inches) and an at-rest, end-to-end length (L2) of 2.54 cm (one inch). The arcuate length corresponds to the length of the first lead portion 204 from end-to-end if the first lead portion 204 were stretched out to eliminate the curved portions. The end-to-end length (L2) corresponds to the length of the first lead portion 204 from its proximal end (at anchor 206) to its distal end (at cuff electrode 202) without accounting for the length of the curved portions between the respective ends of the first lead portion 204. However, in other embodiments, the first lead portion 204 has other lengths greater or less than the above-described dimensions provided that the selected length is substantially greater than an expected degree of movement or range of motion of the target nerve 310 relative to the adjacent non-nerve anatomical structure 330.

In some embodiments, as illustrated in FIG. 6, the second lead portion 208 has an arcuate length (L3) of 2.54 to 30.48 cm (ten to twelve inches) (i.e., including the length of the curved portions and an end-to-end length (i.e., excluding the length of the curved portions) of 17.78 cm (seven inches). However, in other embodiments, the second lead portion 208 has other lengths greater or less than the above-described dimensions provided that the selected length is sufficiently long to compensate for some degree of bodily movement between the point at which anchor 206 is fastened and the location of the IPG 55 (FIG. 1).

It is understood that at least some of these actions of method 300 need not be performed in a particular order.

Embodiments of the present disclosure ensure long term, robust deployment of a cuff electrode on a nerve via a lead system. In some embodiments, this robust engagement is accomplished via placing a relatively long, generally serpentine lead portion between the secured cuff electrode and an anchor secured to a non-nerve anatomical structure close to the cuff electrode and nerve.

While at least one exemplary embodiment has been presented in the foregoing detailed description, it should be appreciated that variations exist. It should also be appreciated that the exemplary embodiment or exemplary embodiments are only examples, and are not intended to limit the scope, applicability, or configuration of the present disclosure in any way. Rather, the foregoing detailed description will provide those skilled in the art with a convenient road map for implementing the exemplary embodiment or exemplary embodiments. It should be understood that various changes can be made in the function and arrangement of elements without departing from the scope of the present disclosure as set forth in the appended claims and the legal equivalents thereof.

## Claims

1. A lead system (80, 200) comprising:
a cuff electrode (90, 100, 202);
a first lead portion (204) extending from the cuff electrode and having a generally serpentine configuration;
a second lead portion (208) having a generally serpentine configuration and having a length (L4) substantially greater than a length (L2) of the first lead portion;
a first anchor (206) interposed between the first lead portion and the second lead portion and configured to be secured relative to a non-nerve bodily structure (330);
has a connect (88, 210) extending proximally from the second lead portion and configured to electrically connect to an implantable pulse generator;
wherein the first lead portion has a length substantially greater than a distance between a nerve, onto which the cuff electrode is attached, and the non-nerve bodily structure onto which the first anchor is secured such that at least a portion of the first lead portion retains the generally serpentine configuration,
wherein the cuff electrode comprises:
an elongate cuff body (101) including:
a base member (120) including a top wall (122);
a first flange member (130) including a first end (131) and a second end (132); and
a pair of resilient arcuate-shaped flange members including a second flange member (134) and a third flange member (150), the respective second and third flange members each including a proximal portion that extends from the base member outwardly and away from the other respective proximal portion and a distal portion biased to extend toward the other respective distal portion, wherein the base member and the second and third flange members define a lumen (140) with the distal portions defining has a substantially re-closable opening (109) of the lumen, wherein at least the second and third flange members extend a length of the cuff body,wherein the first end of the first flange member is secured to an outer surface of the third flange member and in a nerve-engaging position, the first flange member is biased to extend from the secured first end to overlap, and be in releasable contact with, a portion of the third flange member, the substantially re-closable opening, and the second flange member, and wherein the second end of the first flange member is located over the second flange member; and
a row (102) of at least three generally rigid electrode elements (92, 103) embedded within the cuff body and spaced apart from each other along the length of the cuff body.

2. The lead system of claim 1 wherein the generally serpentine configuration of the respective first and second lead portions comprises a generally sinusoidal pattern.

3. The lead system of claim 1 wherein the second lead portion has a length (L4) about seven times the length (L2) of the first lead portion.

4. The lead system of claim 1, wherein the length of the first lead portion corresponds to an arcuate length that includes the length of the curved portion of the generally serpentine configuration.

5. The lead system of claim 1, wherein each electrode element includes:
a generally arcuate first portion (111) extending along an inner surface of at least one of the respective second and third flange members to be exposed at the lumen, wherein the exposed first portion of the electrode element is located generally opposite the substantially re-closable opening of the cuff body; and
a generally straight second portion (110) extending into the base member and away from the first portion at a generally obtuse angle relative to the first portion.

6. The lead system of claim 1, wherein the first flange member extends a length of the cuff body.

7. The lead system of claim 6, wherein the base member (102) includes a bottom wall (124) opposite the top wall (122), a first side wall (126), and a second side wall (128) opposite the first side wall, wherein the bottom wall extends from first side wall to the second side wall.

8. The lead system of claim 1, wherein the base member (102) induces a bottom wall (124) opposite the top wall (122), a first side wall (126), and a second side wall (128) opposite the first side wall, wherein the bottom wall extends from first side wall to the second side wall.

9. The lead system of claim 8, wherein the second and third flange members do not overlap each other.

10. The lead system of claim 1, comprising:
a second anchor connected to the second lead portion and interposed between the second lead portion and the connector, wherein the second anchor is configured to be secured relative to a second non-nerve bodily structure.

11. The lead system of claim 1, wherein, with the cuff electrode secured to a nerve and with the first anchor secured to the non-nerve bodily structure, the cuff electrode and the first anchor are configured to extend in the same vertical plane, and the first lead portion is configured to extend generally perpendicular to the vertical plane while retaining the serpentine configuration.

## Patentansprüche

1. Leitungssystem (80, 200) mit:
einer Manschettenelektrode (90, 100, 202);
einem ersten Leitungsabschnitt (204), der sich von der Manschettenelektrode erstreckt und eine im Allgemeinen serpentinenförmige Konfiguration hat;
einem zweiten Leitungsabschnitt (208), der eine im Allgemeinen serpentinenförmige Konfiguration und eine Länge (L4) hat, die wesentlich größer ist als eine Länge (L2) des ersten Leitungsabschnitts;
einem ersten Anker (206), der sich zwischen dem ersten Leitungsabschnitt und dem zweiten Leitungsabschnitt befindet und dazu konfiguriert ist, bezüglich einer Körperstruktur (330) ohne Nerv befestigt zu werden;
einem Anschluss (88, 210), der sich proximal von dem zweiten Leitungsabschnitt erstreckt und dazu konfiguriert ist, elektrisch an einen implantierbaren Impulsgenerator angeschlossen zu werden;
wobei der erste Leitungsabschnitt eine Länge hat, die wesentlich größer ist als ein Abstand zwischen einem Nerv, an dem die Manschettenelektrode befestigt ist, und der Körperstruktur ohne Nerv, an der der erste Anker befestigt ist, so dass wenigstens ein Teil des ersten Leitungsabschnitts die im Allgemeinen serpentinenförmige Konfiguration beibehält;
wobei die Manschettenelektrode aufweist:
einen länglichen Manschettenkörper (101), der:
ein Basisteil (120), das eine obere Wand (122) enthält;
ein erstes Flanschteil (130), das ein erstes Ende (131) und ein zweites Ende (132) enthält; und
ein Paar biegsamer bogenförmiger Flanschteile aufweist, die ein zweites Flanschteil (134) und ein drittes Flanschteil (150) aufweisen, wobei die jeweiligen zweiten und dritten Flanschteile jeweils einen proximalen Abschnitt aufweisen, der sich von dem Basisteil nach außen und von dem anderen jeweiligen proximalen Abschnitt weg erstreckt, und einen distalen Abschnitt, der vorgespannt ist, um sich in Richtung des anderen jeweiligen distalen Abschnitts zu erstrecken, wobei das Basisteil und das zweite und dritte Flanschteil ein Lumen (140) definieren und die distalen Abschnitte eine im Wesentlichen wiederverschließbare Öffnung (109) des Lumens definieren, wobei sich wenigstens das zweite und das dritte Flanschteil über eine Länge des Manschettenkörpers erstrecken, wobei das erste Ende des ersten Flanschteils an einer Außenfläche des dritten Flanschteils und in einer mit einem Nerv im Eingriff stehenden Position befestigt ist, das erste Flanschteil vorgespannt ist, um sich von dem befestigten ersten Ende zu erstrecken, um einen Abschnitt des dritten Flanschteils, die im Wesentlichen wiederverschließbare Öffnung und das zweite Flanschteil zu überlappen und damit in lösbarem Kontakt zu stehen, und wobei das zweite Ende des ersten Flanschteils über dem zweiten Flanschteil angeordnet ist; und
eine Reihe (102) von wenigstens drei im Allgemeinen starren Elektrodenelementen (92, 103), die in den Manschettenkörper eingebettet und entlang der Länge des Manschettenkörpers voneinander beabstandet sind.

2. Leitungssystem nach Anspruch 1, wobei die im Allgemeinen serpentinenförmige Konfiguration der jeweiligen ersten und zweiten Leitungsabschnitte ein im Wesentlichen sinusförmiges Muster hat.

3. Leitungssystem nach Anspruch 1, wobei der zweite Leitungsabschnitt eine Länge (L4) hat, die ungefähr sieben mal die Länge (L2) des ersten Leitungsabschnitts beträgt.

4. Leitungssystem nach Anspruch 1, wobei die Länge des ersten Leitungsabschnitts einer Bogenlänge entspricht, die die Länge des gebogenen Abschnitts der im Allgemeinen serpentinenförmigen Konfiguration aufweist.

5. Leitungssystem nach Anspruch 1, wobei jedes Elektrodenelement aufweist:
einen im Allgemeinen bogenförmigen ersten Abschnitt (111), der sich entlang einer Innenfläche von wenigstens einem der jeweiligen zweiten und dritten Flanschteile erstreckt, um an dem Lumen freigelegt zu sein, wobei der freigelegte erste Abschnitt des Elektrodenelements im Allgemeinen gegenüber der im Wesentlichen wiederverschließbaren Öffnung des Manschettenkörpers angeordnet ist; und
einen im Allgemeinen geraden zweiten Abschnitt (110), der sich in das Basisteil und von dem ersten Abschnitt in einem im Allgemeinen stumpfen Winkel relativ zum ersten Abschnitt weg erstreckt.

6. Leitungssystem nach Anspruch 1, wobei sich das erste Flanschteil über eine Länge des Manschettenkörpers erstreckt.

7. Leitungssystem nach Anspruch 6, wobei das Basisteil (102) eine untere Wand (124) gegenüber der oberen Wand (122), eine erste Seitenwand (126) und eine zweite Seitenwand (128) gegenüber der ersten Seitenwand aufweist, wobei sich die untere Wand von der ersten Seitenwand zu der zweiten Seitenwand erstreckt.

8. Leitungssystem nach Anspruch 1, wobei das Basisteil (102) eine untere Wand (124) gegenüber der oberen Wand (122), eine erste Seitenwand (126) und eine zweite Seitenwand (128) gegenüber der ersten Seitenwand aufweist, wobei sich die untere Wand von der ersten Seitenwand zu der zweiten Seitenwand erstreckt.

9. Leitungssystem nach Anspruch 8, wobei das erste und das dritte Flanschteil einander nicht überlappen.

10. Leitungssystem nach Anspruch 1 mit:
einem zweiten Anker, der mit dem zweiten Leitungsabschnitt verbunden ist und sich zwischen dem zweiten Leitungsabschnitt und dem Anschluss befindet, wobei der zweite Anker dazu konfiguriert ist, bezüglich einer zweiten Körperstruktur ohne Nerv befestigt zu werden.

11. Leitungssystem nach Anspruch 1, wobei, wenn die Manschettenelektrode an einem Nerv befestigt ist und der erste Anker an der Körperstruktur ohne Nerv befestigt ist, die Manschettenelektrode und der erste Anker dazu konfiguriert sind, sich in derselben vertikalen Ebene zu erstrecken, und der erste Leitungsabschnitt dazu konfiguriert ist, sich im Wesentlichen senkrecht zu der vertikalen Ebene zu erstrecken, während die serpentinenförmige Konfiguration beibehalten wird.

## Revendications

1. Système de dérivation (80, 200) comprenant :
une électrode à manchon (90, 100, 202),
une première partie de dérivation (204) s'étendant à partir de l'électrode à manchon et ayant une configuration généralement en serpentin ;
une seconde partie de dérivation (208) ayant une configuration généralement en serpentin et ayant une longueur (L4) sensiblement plus grande qu'une longueur (L2) de la première partie de dérivation ;
un premier ancrage (206) interposé entre la première partie de dérivation et la seconde partie de dérivation et configuré pour être fixé par rapport à une structure corporelle non nerveuse (330),
un connecteur (88, 210) s'étendant de manière proximale à partir de la seconde partie de dérivation et configuré pour connecter électriquement à un générateur d'impulsions implantable ;
la première partie de dérivation ayant une longueur sensiblement plus grande qu'une distance entre un nerf, sur lequel l'électrode à manchon est fixé, et la structure corporelle non nerveuse sur laquelle le premier ancrage est fixé, de telle sorte qu'au moins une partie de la première partie de dérivation conserve la configuration généralement en serpentin,
dans lequel l'électrode à manchon comprend :
un corps de manchon allongé (101) comprenant :
un élément de base (120) comprenant une paroi supérieure (122),
un premier élément bride (130) comprenant une première extrémité (131) et une seconde extrémité (132), et
une paire d'éléments brides élastiques en forme d'arc comprenant un deuxième élément bride (134) et un troisième élément bride (150), les deuxième et troisième éléments brides respectifs comprenant chacun une partie proximale qui s'étend à partir de l'élément de base vers l'extérieur et à l'opposé de l'autre partie proximale respective et une partie distale sollicitée pour s'étendre vers l'autre partie distale respective, l'élément de base et les deuxième et troisième éléments brides définissant une lumière (140) avec les parties distales définissant une ouverture sensiblement refermable (109) de la lumière, au moins les deuxième et troisième éléments brides s'étendant sur une longueur du corps de manchon, la première extrémité du premier élément bride étant fixée à une surface externe du troisième élément bride et dans une position d'engagement de nerf, le premier élément bride étant sollicité pour s'étendre à partir de la première extrémité fixée pour chevaucher, et être contact libérable avec, une partie du troisième élément bride, de l'ouverture sensiblement refermable et du deuxième élément bride, et la seconde extrémité du premier élément bride étant située sur le deuxième élément bride ; et
une rangée (102) d'au moins trois éléments d'électrode généralement rigides (92, 103) intégrés à l'intérieur du corps de manchon et espacés les uns des autres le long de la longueur du corps de manchon.

2. Système de dérivation selon la revendication 1, dans lequel la configuration généralement en serpentin des première et seconde parties de dérivation respectives comprend un motif généralement sinusoïdal.

3. Système de dérivation selon la revendication 1, dans lequel la seconde partie de dérivation has a une longueur (L4) d'environ sept fois la longueur (L2) de la première partie de dérivation.

4. Système de dérivation selon la revendication 1, dans lequel la longueur de la première partie de dérivation correspond à une longueur arquée qui comprend la longueur de la partie incurvée de la configuration généralement en serpentin.

5. Système de dérivation selon la revendication 1, dans lequel chaque élément d'électrode comprend :
une première partie généralement arquée (111) s'étendant le long d'une surface interne d'au moins l'un des deuxième et troisième éléments brides respectifs pour être exposée au niveau de la lumière, la première partie exposée de l'élément d'électrode étant située généralement opposée à l'ouverture sensiblement refermable du corps de manchon ; et
une seconde partie généralement rectiligne (110) s'étendant dans l'élément de base et à l'opposé de la première partie à un angle généralement obtus par rapport à la première partie.

6. Système de dérivation selon la revendication 1, dans lequel le premier élément bride s'étend sur une longueur du corps de manchon.

7. Système de dérivation selon la revendication 6, dans lequel l'élément de base (102) comprend une paroi inférieure (124) opposée à la paroi supérieure (122), une première paroi latérale (126) et une seconde paroi latérale (128) opposée à la première paroi latérale, la paroi inférieure s'étendant de la première paroi latérale à la seconde paroi latérale.

8. Système de dérivation selon la revendication 1, dans lequel l'élément de base (102) comprend une paroi inférieure (124) opposée à la paroi inférieure (122), une première paroi latérale (126) et une seconde paroi latérale (128) opposée à la première paroi latérale, la paroi inférieure s'étendant de la première paroi latérale à la seconde paroi latérale.

9. Système de dérivation selon la revendication 8, dans lequel les deuxième et troisième éléments brides ne se chevauchent pas l'un l'autre.

10. Système de dérivation selon la revendication 1, comprenant :
un second ancrage connecté à la seconde partie de dérivation et interposé entre la seconde partie de dérivation et le connecteur, le second ancrage étant configuré pour être fixé par rapport à une seconde structure corporelle non nerveuse.

11. Système de dérivation selon la revendication 1, dans lequel, avec l'électrode à manchon fixé à un nerf et avec le premier ancrage fixé à la structure corporelle non nerveuse, l'électrode à manchon et le premier ancrage sont configurés pour s'étendre dans le même plan vertical, et la première partie de dérivation est configurée pour s'étendre généralement perpendiculairement au plan vertical tout en conservant la configuration en serpentin.
